# EUROPEAN PATENT APPLICATION

(11) **EP 4 603 025 A1**
(43) Date of publication of application: **20.08.2025**
(21) Application number: 23885290.9
(22) Date of filing: 07.06.2023
(51) Int. Cl.: A61B 10/00, G01N 21/64

(54) **LYMPHATIC SYSTEM EXAMINATION DEVICE**

(30) Priority: 04.11.2022 JP 2022177498
(71) Applicant: Hamamatsu Photonics K.K., Hamamatsu-shi, Shizuoka 435-8558 (JP)
(72) Inventor: SHIKAYAMA, Takahiro, Hamamatsu-shi, Shizuoka 435-8558 (JP); MURAKOSHI, Takahiro, Hamamatsu-shi, Shizuoka 435-8558 (JP); SATO, Takayuki, Hamamatsu-shi, Shizuoka 435-8558 (JP); MIZUNO, Toshihiko, Hamamatsu-shi, Shizuoka 435-8558 (JP)
(74) Representative: Hoffmann Eitle
(86) International application number: PCT/JP2023/021077
(87) International publication number: WO 2024/095516

(57) **Abstract**

A lymphatic system examination device includes a main body portion including a placement portion on which a subject stands, and configured to position a lower body of a subject in an imaging region, an illumination unit configured to irradiate the lower body with excitation light; and an imaging unit configured to detect fluorescence emitted from the lower body. The illumination unit and the imaging unit are arranged on one side in a first horizontal direction with respect to the imaging region. The main body portion further includes a restricting portion. The restricting portion is disposed along the upper end portion of the imaging region and restricts movement of the subject to one side in the first horizontal direction.

## Description

### Technical Field

The present disclosure relates to a lymphatic system examination device.

### Background Art

The lymphatic system is a network that functions as a circulatory system of lymphatic fluid and is composed of lymph nodes, lymphatic vessels, and the like. As an examination method for diagnosing a disease of the lymphatic system (for example, lymphedema), a method is known in which a predetermined part of a subject into which a fluorescent dye such as indocyanine green is injected into the lymphatic system is irradiated with excitation light, and fluorescence emitted from the predetermined part in response to the irradiation of the excitation light is detected to acquire a fluorescent image of the predetermined part (see, for example, Patent Literature 1).

### Citation List

### Patent Literature

Patent Literature 1: Japanese Unexamined Patent Publication No. 2019-118803

### Summary of Invention

### Technical Problem

The device used for the examination as described above has a problem that the irradiation range of the excitation light and the detection range of the fluorescence are limited to narrow ranges, and it is difficult to efficiently examine the lymphatic system in the lower body of the subject.

An object of the present disclosure is to provide a lymphatic system examination device suitable for examination of a lymphatic system in the lower body of a subject.

### Solution to Problem

A lymphatic system examination device according to an aspect of the present disclosure is [1] "a lymphatic system examination device including: a main body portion including a placement portion on which a subject in which a fluorescent dye is injected into a lymphatic system stands, and configured to position a lower body of the subject in an imaging region on the placement portion; an illumination unit configured to irradiate the lower body with excitation light; and an imaging unit configured to detect fluorescence emitted from the lower body in response to irradiation of the excitation light, in which the illumination unit and the imaging unit are arranged on one side in a first horizontal direction with respect to the imaging region, the main body portion further includes a restricting portion, and the restricting portion is disposed along the upper end portion of the imaging region and restricts movement of the subject to one side in the first horizontal direction".

In the lymphatic system examination device according to [1], the illumination unit and the imaging unit are arranged on one side in the first horizontal direction with respect to the imaging region where the lower body of the subject is positioned, and the movement of the subject to one side in the first horizontal direction is restricted by the restricting portion disposed along the upper end portion of the imaging region. As a result, the distance between the illumination unit and the lower body of the subject and the distance between the imaging unit and the lower body of the subject can be maintained constant, and shaking of the lower body of the subject can be suppressed. Therefore, according to the lymphatic system examination device according to [1], it is possible to acquire a fluorescent image of the lower body appropriate for examination of the lymphatic system in the lower body of the subject.

The lymphatic system examination device according to an aspect of the present disclosure may be [2] "the lymphatic system examination device according to [1], further including: a first plate for luminance calibration, the first plate disposed on an external surface on the imaging unit side in the restricting portion extending in a second horizontal direction perpendicular to the first horizontal direction". According to the lymphatic system examination device according to [2], it is possible to adjust the luminance of the fluorescent image for each acquired fluorescent image.

The lymphatic system examination device according to an aspect of the present disclosure may be [3] "the lymphatic system examination device according to [1] or [2], further including: a scale disposed on an external surface on the imaging unit side in the restricting portion extending in a second horizontal direction perpendicular to the first horizontal direction". According to the lymphatic system examination device according to [3], a size of each portion of the lower body can be grasped from the acquired fluorescent image.

The lymphatic system examination device according to an aspect of the present disclosure may be [4] "the lymphatic system examination device according to any one of [1] to [3], further including: a second plate for luminance calibration, the second plate being detachable from the main body portion, in which the second plate is disposed in the main body portion so as to be positioned in the imaging region in a state of being in contact with the restricting portion from a side opposite to the imaging unit". According to the lymphatic system examination device according to [4], it is possible to adjust the luminance of the fluorescent image for each acquired fluorescent image.

The lymphatic system examination device according to an aspect of the present disclosure may be [5] "the lymphatic system examination device according to any one of [1] to [4], further including: a distance sensor disposed on the one side in the first horizontal direction with respect to the imaging region and configured to measure a distance to the lower body positioned in the imaging region". According to the lymphatic system examination device according to [5], the position of the lower body of the subject in the first horizontal direction can be accurately grasped.

### Advantageous Effects of Invention

According to the present disclosure, it is possible to provide a lymphatic system examination device suitable for examination of a lymphatic system in the lower body of a subject.

### Brief Description of Drawings

FIG. 1 is a perspective view of a lymphatic system examination device according to an embodiment.
FIG. 2 is a perspective view of a device main body illustrated in FIG. 1.
FIG. 3 is a cross-sectional view of the device main body taken along line III-III illustrated in FIG. 2.
FIG. 4 is a cross-sectional view of the device main body taken along line IV-IV illustrated in FIG. 3.
FIG. 5 is a cross-sectional view of the device main body taken along line V-V illustrated in FIG. 3.
FIG. 6 is a cross-sectional view of a restricting portion illustrated in FIG. 2.
FIG. 7 is a cross-sectional view of the device main body in which a second plate for luminance calibration is disposed.
FIG. 8 is a diagram illustrating a visible-light image of an object.
FIG. 9 is a diagram illustrating a display unit on which a plurality of types of fluorescent images of the object illustrated in FIG. 8 are displayed.
FIG. 10 is a plan view of a device main body of a modification.

### Description of Embodiments

Hereinafter, embodiments of the present disclosure are described in detail with reference to the accompanying drawings. Note that, in each drawing, the same or corresponding parts are denoted by the same reference signs, and redundant description is omitted.

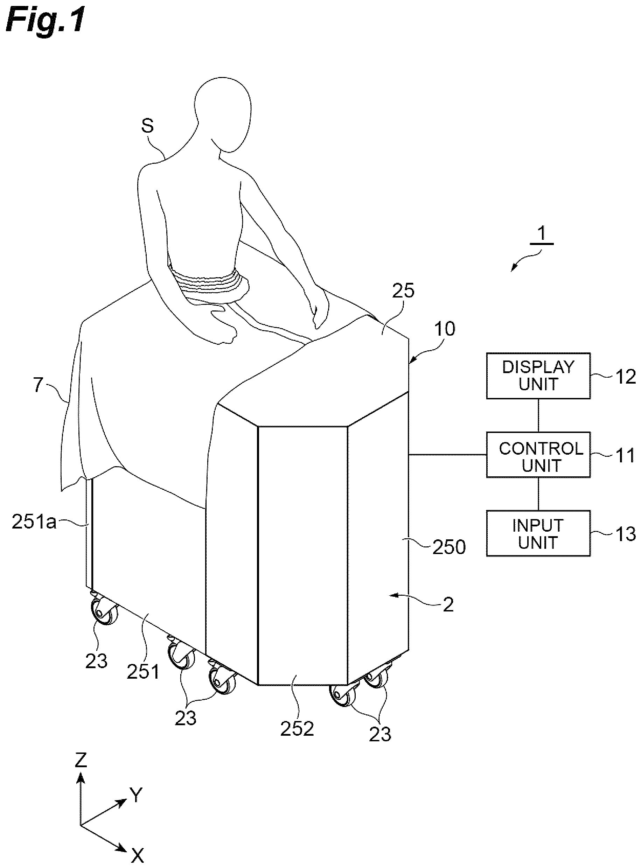

A lymphatic system examination device 1 illustrated in FIG. 1 is a device that examines the lymphatic system in the lower body of a subject S in order to diagnose a disease (for example, lymphedema) of the lymphatic system. More specifically, the lymphatic system examination device 1 is a device that irradiates the lower body of the subject S in which a fluorescent dye such as indocyanine green is injected into the lymphatic system with excitation light and detects fluorescence emitted from the lower body in response to the irradiation of the excitation light, thereby acquiring a fluorescent image of the lower body. Hereinafter, the vertical direction is referred to as a Z direction, the first horizontal direction is referred to as an X direction, and the second horizontal direction perpendicular to the first horizontal direction is referred to as a Y direction.

As illustrated in FIG. 1, the lymphatic system examination device 1 includes a device main body 10, a control unit 11, a display unit 12, and an input unit 13. The control unit 11 is composed of a processing unit and a storage unit. The processing unit of the control unit 11 is a computer device that is composed of a processor, a memory, a storage, a communication device, and the like and processes various items of data by executing software (program). The storage unit of the control unit 11 is a hard disk or the like and stores various items of data. The display unit 12 is a display or the like and displays various items of data to the operator. The input unit 13 is a mouse, a keyboard, or the like and receives inputs of various items of data from an operator.

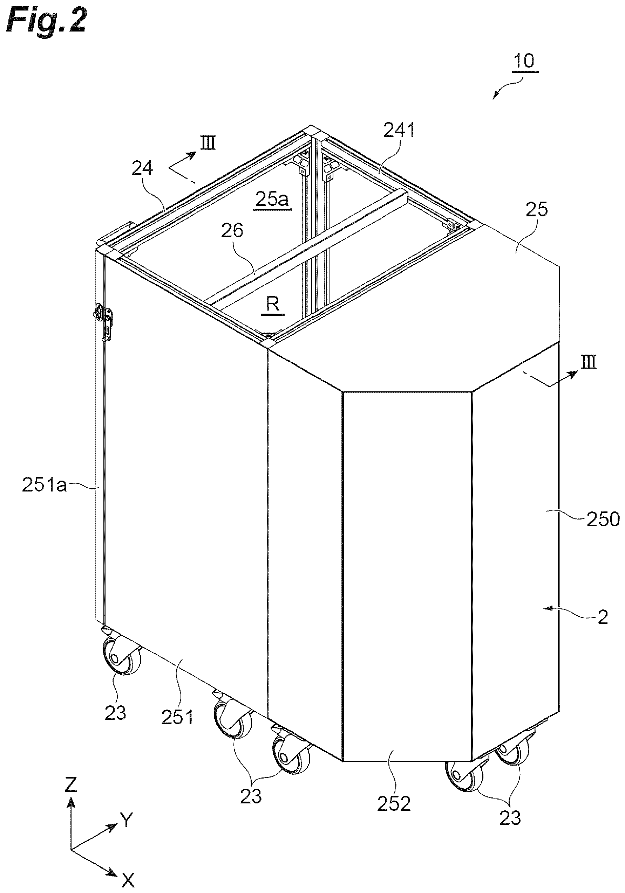

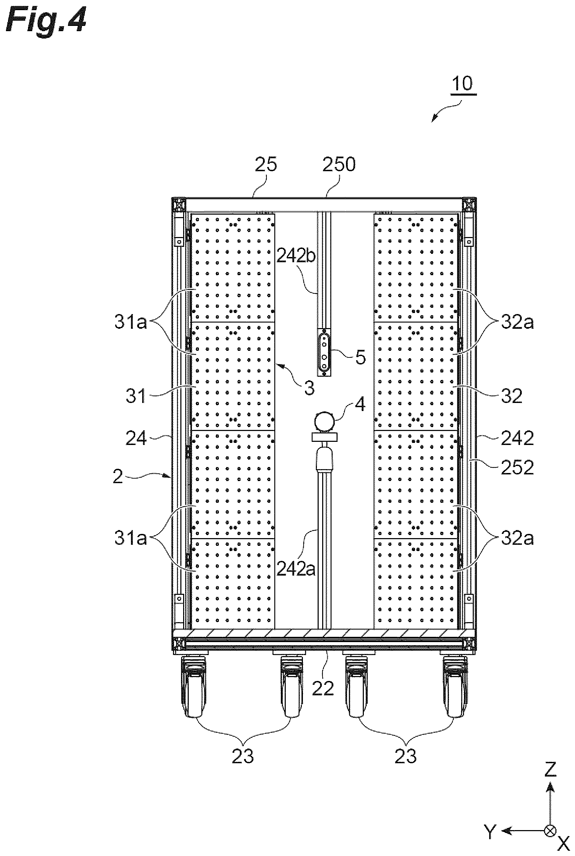

As illustrated in FIGS. 2, 3, 4, and 5, the device main body 10 includes a main body portion 2. The main body portion 2 includes a placement portion 21 and a support portion 22. In the main body portion 2, the placement portion 21 is a portion on which the subject S stands, and the support portion 22 is a portion disposed on one side in the X direction with respect to the placement portion 21. The main body portion 2 is positioned in a lower body L (that is, the portion from the lower abdomen to the toe) of the subject S in the imaging region R on the placement portion 21. A plurality of casters 23 are attached to lower surfaces of the placement portion 21 and the support portion 22. This enables smooth movement of the device main body 10.

The main body portion 2 further includes a frame 24 and a wall portion 25. The frame 24 is composed of a first frame 241 and a second frame 242. The first frame 241 is attached to the placement portion 21, and the second frame 242 is attached to the support portion 22. The wall portion 25 is composed of a first wall portion 251 and a second wall portion 252. The first wall portion 251 is attached to the placement portion 21 via the first frame 241, and the second wall portion 252 is attached to the support portion 22 via the second frame 242. The unit composed of the support portion 22, the second frame 242, and the second wall portion 252 is detachable from the unit composed of the placement portion 21, the first frame 241, and the first wall portion 251. As an example, the width of each unit in the X direction and the width of each unit in the Y direction are 70 cm or less. Accordingly, portability of each unit can be improved.

The wall portion 25 configures the housing 250 on the placement portion 21 and the support portion 22. The housing 250 defines the imaging region R on the placement portion 21. In the housing 250, an opening 25a is formed so as to face the placement portion 21 across the imaging region R. The waist portion of the subject S is positioned inside the opening 25a. That is, the opening 25a in which the subject S is disposed is formed in the wall portion 25. The inner surface of the housing 250 is, for example, black. Note that, in the first wall portion 251, a door is composed of a wall portion 251a on a side opposite to the second wall portion 252. Thus, the subject S can get on and off the placement portion 21.

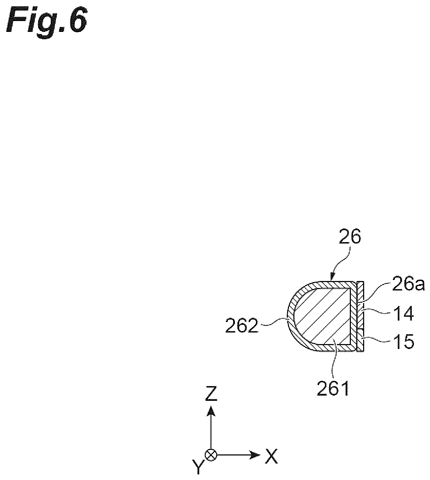

The main body portion 2 further includes a restricting portion 26. The restricting portion 26 is disposed along an upper end portion Ra of the imaging region R. The restricting portion 26 restricts the movement of the subject S to one side (support portion 22 side) in the X direction. In the present embodiment, the restricting portion 26 extends in the Y direction along the upper end portion Ra of the imaging region R and is stretched over the first frame 241 in a state of crossing the opening 25a. As illustrated in FIG. 6, the restricting portion 26 is composed of a bar material 261 and a cushion material 262. The bar material 261 is a core material stretched to the first frame 241. The cushion material 262 is an elastic material wound around the bar material 261.

As illustrated in FIGS. 2, 3, 4, and 5, the device main body 10 further includes an illumination unit 3, an imaging unit 4, and a distance sensor 5. The illumination unit 3, the imaging unit 4, and the distance sensor 5 are arranged on one side (support portion 22 side) in the X direction with respect to the imaging region R. The housing 250 accommodates the illumination unit 3, the imaging unit 4, and the distance sensor 5 on the support portion 22.

The illumination unit 3 irradiates the lower body L with excitation light. The illumination unit 3 includes a first light emitting region 31 and a second light emitting region 32 arranged in a state of being separated from each other in the Y direction. Each of the first light emitting region 31 and the second light emitting region 32 extends in the Z direction in a state of facing the imaging region R. The first light emitting region 31 and the second light emitting region 32 are arranged so as to be away from each other in the Y direction as approaching the imaging region R in the X direction. The first light emitting region 31 includes a plurality of first light emitting portions 31a, and the second light emitting region 32 includes a plurality of second light emitting portions 32a. The illumination unit 3 has a function of adjusting the light emission intensity of each of the first light emitting portions 31a and the light emission intensity of each of the second light emitting portions 32a. Each of the first light emitting portion 31a and the second light emitting portion 32a is composed of, for example, a plurality of LEDs that emit light having a center wavelength of 735 nm. The illumination unit 3 is attached to the second frame 242. That is, the illumination unit 3 is supported by the support portion 22 via the second frame 242.

The imaging unit 4 detects fluorescence emitted from the lower body L in response to irradiation with excitation light. The imaging unit 4 faces the imaging region R across a region between the first light emitting region 31 and the second light emitting region 32. The imaging unit 4 is disposed on one side (a side opposite to the imaging region R) in the X direction with respect to the illumination unit 3. That is, the illumination unit 3 is positioned between the imaging region R and the imaging unit 4 in the X direction. The imaging unit 4 is positioned in the center of the imaging region R when viewed from the X direction. In the present embodiment, the imaging unit 4 is attached to a bar material 242a which is a member extending upward from the support portion 22 side in the second frame 242. That is, the imaging unit 4 is supported by the support portion 22 via the second frame 242.

As an example, the imaging unit 4 is composed of a photodetector, a filter disposed in a preceding stage of the photodetector, a lens disposed in a preceding stage of the filter, and the like. As the photodetector, an area sensor (for example, CCD and CMOS) having sensitivity to fluorescence (for example, light in the near-infrared region) is used. As the filter, a vapor deposition filter that selectively transmits fluorescence is used. The filter may include an absorption filter to remove the influence of oblique light. As the lens, a wide-angle lens is used so that an image of the entire lower body L can be captured.

The distance sensor 5 measures a distance to the lower body L positioned in the imaging region R. The distance sensor 5 may be, for example, an ultrasonic distance sensor or an optical distance sensor such as a triangulation method or a ToF method. The distance sensor 5 faces the imaging region R across a region between the first light emitting region 31 and the second light emitting region 32. The distance sensor 5 is disposed on one side (a side opposite to the imaging region R) in the X direction with respect to the illumination unit 3. The distance sensor 5 is positioned in the center of the imaging region R in the Y direction when viewed from the X direction. In the present embodiment, the distance sensor 5 is attached to a bar material 242b which is a member extending downward from the support portion 22 side in the second frame 242. That is, the distance sensor 5 is supported by the support portion 22 via the second frame 242. Note that, based on the distance measured by the distance sensor 5, the light emission intensity of the illumination unit 3 may be adjusted so that the intensity of the excitation light with which the lower body L is irradiated becomes uniform. Such adjustment of the light emission intensity of the illumination unit 3 may be performed based on the intensity of the excitation light detected by a photodetector provided separately.

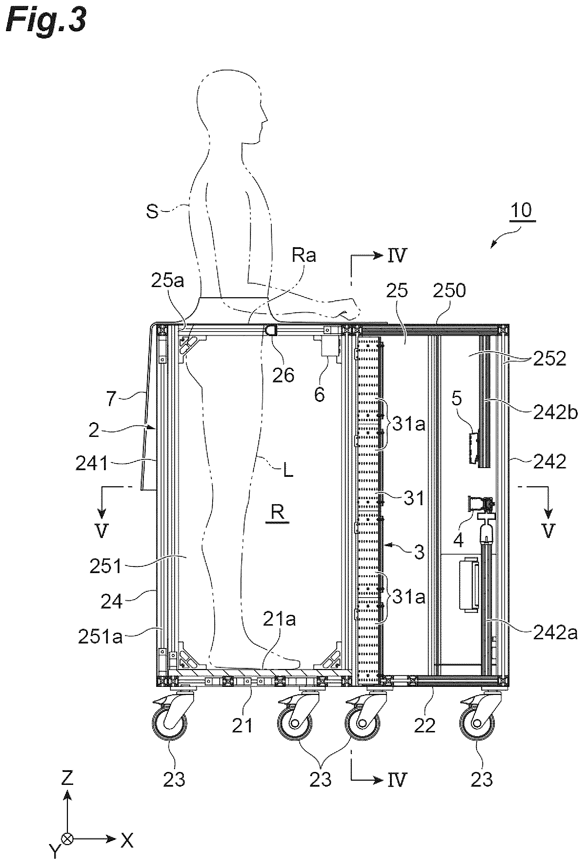

As illustrated in FIG. 3, the device main body 10 includes a camera 6. The camera 6 faces the placement portion 21 and is attached to the first frame 241 so as not to enter the field of view of the imaging unit 4. The housing 250 accommodates the camera 6 on the placement portion 21. The space in the housing 250 is set as a dark room by a cover 7 that covers the opening 25a in a state of being attached to the subject S. Since the camera 6 captures an image of the feet of the subject S in the space in the housing 250 that is set as a dark room, the camera 6 may be composed of an illumination device and an image capturing device or may be configured only with the image capturing device such as an infrared camera. The image acquired by the camera 6 is displayed by the display unit 12 to be shown to the subject S. Note that the camera 6 may be attached to the restricting portion 26 or may be attached to the second frame 242. When the camera 6 is composed of an illumination device and an imaging device, the illumination device may be separated from the image capturing device.

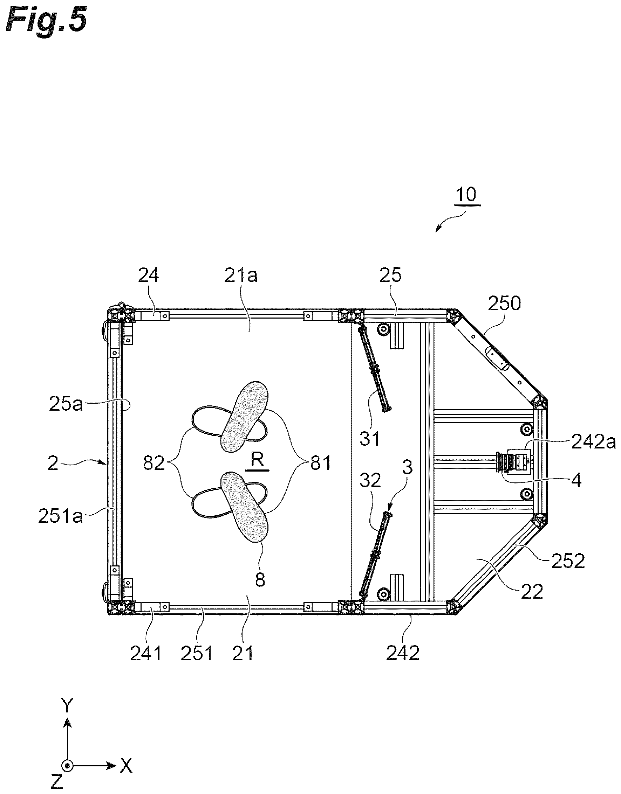

As illustrated in FIG. 5, the placement portion 21 is provided with a positioning portion 8 that indicates the standing position of the subject S. In the present embodiment, the positioning portion 8 includes a foot rest 81 in a state where the subject S faces the imaging unit 4 side, and a foot rest 82 in a state where the subject S faces the side opposite to the imaging unit 4. Each of the foot rests 81 and 82 is a mark formed on the placement surface 21a of the placement portion 21. The image of the positioning portion 8 is captured by the camera 6.

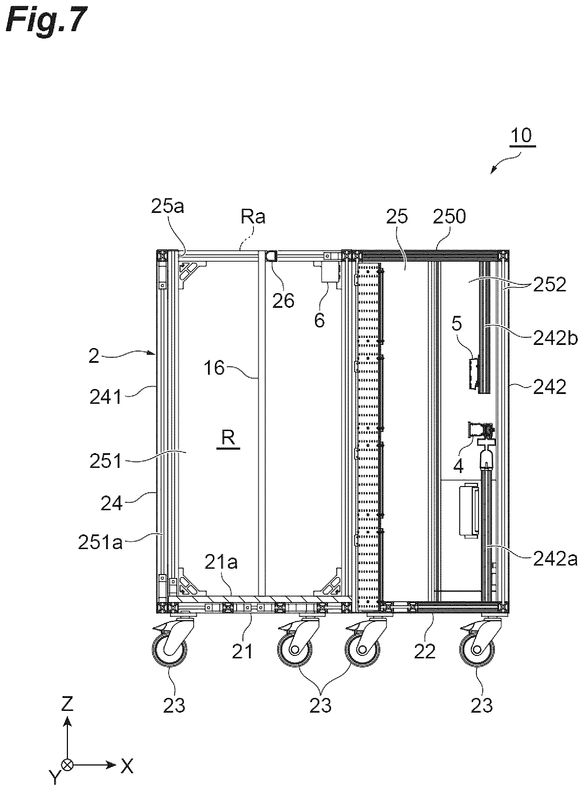

As illustrated in FIG. 6, a first plate 14 and a scale 15 are attached to an external surface 26a of the restricting portion 26 on the imaging unit 4 side. The first plate 14 is a plate for luminance calibration. As an example, the first plate 14 is composed of a plurality of types of members (for example, a cloth and an acrylic plate) having different intensities of fluorescence emitted in response to irradiation with excitation light. The scale 15 is a member having a gradation indicating a length. As illustrated in FIG. 7, a second plate 16 is detachable from the main body portion 2. The second plate 16 is disposed on the main body portion 2 so as to be positioned in the imaging region R in a state of being in contact with the restricting portion 26 from the side opposite to the imaging unit 4. The second plate 16 is a plate for luminance calibration and is, for example, an acrylic plate.

In the lymphatic system examination device 1 configured as described above, the control unit 11 is electrically connected to each of the illumination unit 3, the imaging unit 4, the distance sensor 5, the camera 6, the display unit 12, and the input unit 13. The control unit 11 controls the illumination unit 3 to emit the excitation light as a flash in accordance with the image capturing timing of the imaging unit 4. As a result, it is possible to suppress a change in the light amount of the excitation light applied to the lower body L at the time of capturing an image of the lower body L. The control unit 11 generates a fluorescent image of the lower body L based on the fluorescence detected by the imaging unit 4, and controls the display unit 12 to display the fluorescent image of the lower body L. The control unit 11 stores the fluorescent image of the lower body L generated in the past for the subject S and can control the display unit 12 so that the fluorescent image of the lower body L generated in the past and the fluorescent image of the lower body L generated newly are displayed side by side. In addition, the control unit 11 can control the display unit 12 so that the visible-light image of the lower body L and the fluorescent image of the lower body L are displayed side by side. Furthermore, the control unit 11 can control the display unit 12 so that a part of at least one image of the lower body L (visible-light image, fluorescent image) is enlarged based on the instruction input to the control unit 11 via the input unit 13.

In the present embodiment, the control unit 11 controls at least one of the illumination unit 3 and the imaging unit 4 so as to change at least one of the light emission time of the illumination unit 3 and the exposure time of the imaging unit 4, thereby generating a plurality of types of fluorescent images as the fluorescent image of the lower body L and controlling the display unit 12 so that the plurality of types of fluorescent images are displayed side by side. That is, in the lymphatic system examination device 1, a lymphatic system examination method including a step of generating a plurality of types of fluorescent images as the fluorescent image of the lower body L by controlling at least one of the illumination unit 3 and the imaging unit 4 so that at least one of the light emission time of the illumination unit 3 and the exposure time of the imaging unit 4 is changed (that is, the exposure amount in the imaging unit 4 is changed), and a step of controlling the display unit 12 so that the plurality of types of fluorescent images are displayed side by side is performed.

In the lymphatic system examination method, the control unit 11 generates each of the plurality of types of fluorescent images in a time of one second or less (that is, the time is one second or less after the image capturing instruction is input to the control unit 11 via the input unit 13). Note that the exposure amount in the imaging unit 4 may be changed by changing at least one of the light emission time of the illumination unit 3, the light emission intensity of the illumination unit 3, the exposure time of the imaging unit 4, the sensor gain of the imaging unit 4, and the lens diaphragm of the imaging unit 4.

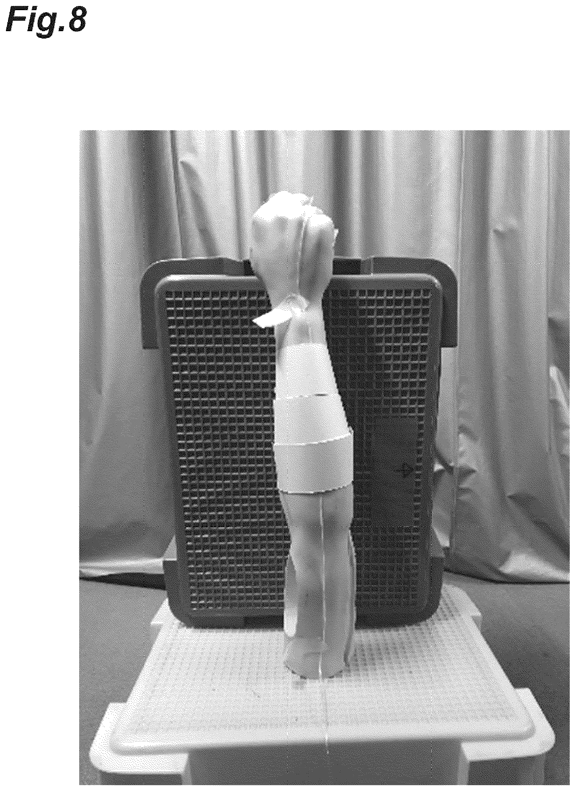

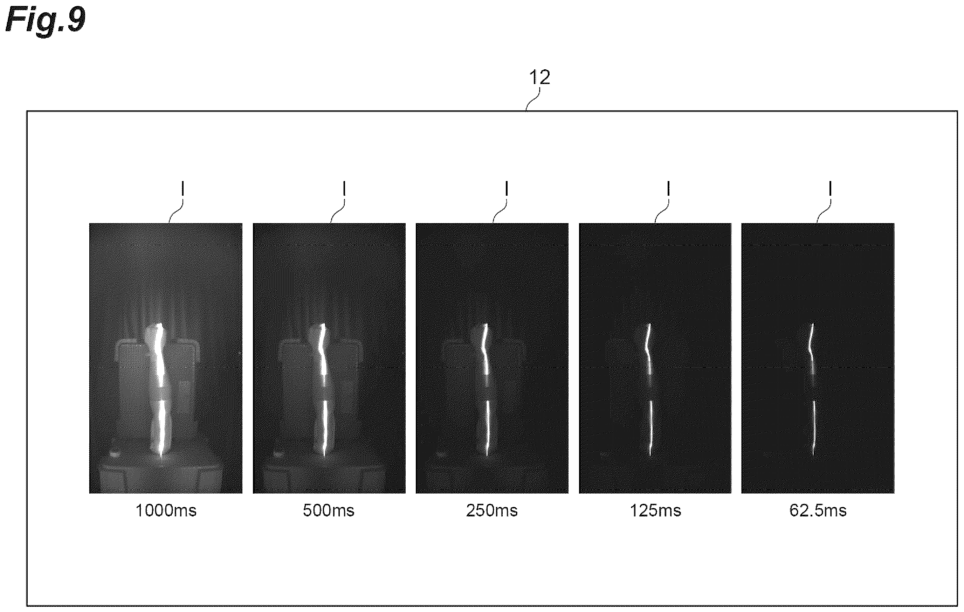

FIG. 8 is a diagram illustrating a visible-light image of the object, and FIG. 9 is a diagram illustrating the display unit 12 on which a plurality of types of fluorescent images I for the object illustrated in FIG. 8 are displayed. The object illustrated in FIG. 8 is obtained by winding white paper around an arm model to which a phosphor extending in the vertical direction is adhered. The white paper covers the phosphor in a single, double, and triple state in the order from the top. The plurality of types of fluorescent images I illustrated in FIG. 9 are images generated with the light emission time of the illumination unit 3 and the exposure time of the imaging unit 4 set to 1000 ms, 500 ms, 250 ms, 125 ms, and 62.5 ms. When the light emission time of the illumination unit 3 and the exposure time of the imaging unit 4 are 500 ms or more, the portion of the single white paper is observed to be considerably thicker than the actual phosphor due to the scattering of the fluorescence. When the light emission time of the illumination unit 3 and the exposure time of the imaging unit 4 are 125 ms or more, the phosphor can be visually recognized in the portion of the double white paper. When the light emission time of the illumination unit 3 and the exposure time of the imaging unit 4 are 500 ms or more, the phosphor can be visually recognized in the portion of the triple white paper. From the above, it can be seen that the state of the lymphatic system in the lower body L can be appropriately grasped by adjusting at least one of the light emission time of the illumination unit 3 and the exposure time of the imaging unit 4.

As described above, in the lymphatic system examination device 1, each of the first light emitting region 31 and the second light emitting region 32 in the illumination unit 3 extends in the Z direction in a state of facing the imaging region R, and the imaging unit 4 faces the imaging region R across a region between the first light emitting region 31 and the second light emitting region 32. As a result, the lower body L of the subject S positioned in the imaging region R can be uniformly irradiated with the excitation light, and the fluorescence emitted from the lower body L in response to the uniform irradiation of the excitation light can be detected. Therefore, according to the lymphatic system examination device 1, it is possible to acquire a fluorescent image of the lower body L appropriate for examination of the lymphatic system in the lower body L of the subject S.

In the lymphatic system examination device 1, the imaging unit 4 is positioned at the center of the imaging region R when viewed from the X direction. This makes it possible to suppress occurrence of distortion in the acquired fluorescent image.

In the lymphatic system examination device 1, the illumination unit 3 is positioned between the imaging region R and the imaging unit 4 in the X direction. As a result, the lower body L of the subject S positioned in the imaging region R can be more uniformly irradiated with the excitation light.

In the lymphatic system examination device 1, the illumination unit 3 has a function of adjusting the light emission intensity of each of the first light emitting portions 31a and the light emission intensity of each of the second light emitting portions 32a. As a result, in response to the shape and the like of the lower body L of the subject S positioned in the imaging region R, the lower body L can be uniformly irradiated with the excitation light.

In the lymphatic system examination device 1, the first light emitting region 31 and the second light emitting region 32 are arranged so as to be away from each other in the Y direction as approaching the imaging region R in the X direction. As a result, the lower body L of the subject S positioned in the imaging region R can be more uniformly irradiated with the excitation light.

In addition, in the lymphatic system examination device 1, the illumination unit 3 and the imaging unit 4 are arranged on one side in the X direction with respect to the imaging region R where the lower body L of the subject S is positioned, and the movement of the subject S to one side in the X direction is restricted by the restricting portion 26 arranged along the upper end portion Ra of the imaging region R. As a result, the distance between the illumination unit 3 and the lower body L of the subject S and the distance between the imaging unit 4 and the lower body L of the subject S can be maintained constant, and shaking of the lower body L of the subject S can be suppressed. Therefore, according to the lymphatic system examination device 1, it is possible to acquire a fluorescent image of the lower body L appropriate for examination of the lymphatic system in the lower body L of the subject S.

In the lymphatic system examination device 1, the restricting portion 26 extends in the Y direction, and the first plate 14 for luminance calibration is disposed on the external surface 26a of the restricting portion 26 on the imaging unit 4 side. As a result, the luminance of the fluorescent image can be adjusted for each acquired fluorescent image.

In the lymphatic system examination device 1, the restricting portion 26 extends in the Y direction, and the scale 15 is disposed on the external surface 26a of the restricting portion 26 on the imaging unit 4 side. As a result, the size of each part of the lower body L can be grasped from the acquired fluorescent image.

In the lymphatic system examination device 1, the second plate 16 for luminance calibration is disposed on the main body portion 2 so as to be positioned in the imaging region R in a state of being in contact with the restricting portion 26 from the side opposite to the imaging unit 4. As a result, the luminance of the fluorescent image can be adjusted for each lymphatic system examination device 1.

In the lymphatic system examination device 1, the distance sensor 5 is disposed on one side in the X direction with respect to the imaging region R. As a result, the position of the lower body L of the subject S in the X direction can be accurately grasped.

In addition, in the lymphatic system examination device 1 (and the above-described lymphatic system examination method), by changing at least one of the light emission time of the illumination unit 3 and the exposure time of the imaging unit 4, a plurality of types of fluorescent images are generated as the fluorescent image of the lower body L of the subject S, and the plurality of types of fluorescent images are displayed side by side. Although the intensity of fluorescence changes in response to the state of the lymphatic system in the lower body L, the state of the lymphatic system in the lower body L can be appropriately grasped from the plurality of types of fluorescent images generated and displayed as described above. Therefore, according to the lymphatic system examination device 1 (and the above-described lymphatic system examination method), the examination of the lymphatic system in the lower body L of the subject S can be appropriately performed.

In the lymphatic system examination device 1, the control unit 11 generates each of the plurality of types of fluorescent images during the time of one second or less. As a result, it is possible to acquire a plurality of types of fluorescent images while shaking of the lower body L of the subject S is suppressed.

In the lymphatic system examination device 1, the control unit 11 controls the illumination unit 3 so as to emit the excitation light as a flash. As a result, it is possible to acquire a plurality of types of fluorescent images while change of the light amount of the excitation light applied to the lower body L of the subject S is suppressed.

In lymphatic system examination device 1, the control unit 11 stores the fluorescent image of the lower body L generated in the past for the subject S and controls the display unit 12 so that the fluorescent image of the lower body L generated in the past and the fluorescent image of the lower body L generated newly are displayed side by side. This makes it possible to appropriately grasp the temporal change in the state of the lymphatic system in the lower body L of the subject S.

In the lymphatic system examination device 1, the positioning portion 8 indicating the standing position of the subject S is provided in the placement portion 21 on which the subject S stands. As a result, the distance between the illumination unit 3 and the lower body L of the subject S and the distance between the imaging unit 4 and the lower body L of the subject S can be maintained constant, and the direction of both feet of the subject S with respect to the illumination unit 3 and the imaging unit 4 can be matched to a predetermined direction. Therefore, according to the lymphatic system examination device 1, it is possible to acquire a fluorescent image of the lower body L appropriate for examination of the lymphatic system in the lower body L of the subject S.

In the lymphatic system examination device 1, the positioning portion 8 includes the foot rest 81 in a state where the subject S faces the imaging unit 4 side, and the foot rest 82 in a state where the subject S faces the side opposite to the imaging unit 4. As a result, in each of the state in which the subject S faces the imaging unit 4 side and the state in which the subject S faces the side opposite to the imaging unit 4, the direction of both feet of the subject S with respect to the illumination unit 3 and the imaging unit 4 can be matched to a predetermined direction.

In the lymphatic system examination device 1, the wall portion 25 configures the housing 250 that defines the imaging region R and accommodates the illumination unit 3 and the imaging unit 4, and the opening 25a in which the subject S is disposed is formed in the wall portion 25. As a result, it is possible to acquire the fluorescent image of the lower body L of the subject S while the influence of the disturbance light is suppressed.

In the lymphatic system examination device 1, the cover 7 attached to the subject S covers the opening 25a. As a result, it is possible to acquire the fluorescent image of the lower body L of the subject S while the influence of the disturbance light is more reliably suppressed.

In the lymphatic system examination device 1, the support portion 22 and the second wall portion 252 are detachable from the placement portion 21 and the first wall portion 251. Accordingly, portability of the lymphatic system examination device 1 can be improved.

In the lymphatic system examination device 1, the image of the positioning portion 8 is captured by the camera 6. As a result, even when it is difficult for the subject S to directly visually recognize the positioning portion 8, the subject S can match the standing position with the positioning portion 8 while visually recognizing the image acquired by the camera 6.

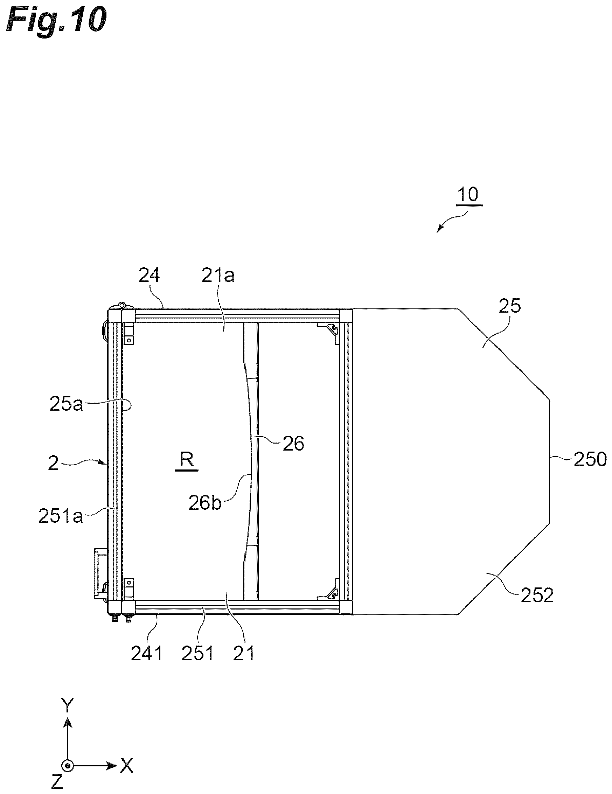

The present disclosure is not limited to the above embodiments. For example, as illustrated in FIG. 10, an external surface 26b of the restricting portion 26 on the side opposite to the imaging unit 4 (see FIG. 3) may be a curved surface curved along the lower abdomen of the subject S (that is, so as to be recessed toward the imaging unit 4 side). Also, the positioning portion 8 may be a recess or a protrusion provided on the placement surface 21a so as to match the foot rests of the subject S instead of the foot rests 81 and 82 or together with the foot rests 81 and 82. At least one of the illumination unit 3, the imaging unit 4, the distance sensor 5, the camera 6, and the restricting portion 26 may be attached to the frame 24 so as to enable at least one of position adjustment and angle adjustment.

### Reference Signs List

- 1: lymphatic system examination device
- 2: main body portion
- 3: illumination unit
- 4: imaging unit
- 5: distance sensor
- 6: camera
- 7: cover
- 8: positioning portion
- 11: control unit
- 12: display unit
- 14: first plate
- 15: scale
- 16: second plate
- 21: placement portion
- 22: support portion
- 25: wall portion
- 25a: opening
- 26: restricting portion
- 26a: external surface
- 31: first light emitting region
- 31a: first light emitting portion
- 32: second light emitting region
- 32a: second light emitting portion
- 81, 82: foot rest
- 250: housing
- 251: first wall portion
- 252: second wall portion
- L: lower body
- R: imaging region
- Ra: upper end portion
- S: subject

## Claims

1. A lymphatic system examination device comprising:
a main body portion including a placement portion on which a subject in which a fluorescent dye is injected into a lymphatic system stands, and configured to position a lower body of the subject in an imaging region on the placement portion;
an illumination unit configured to irradiate the lower body with excitation light; and
an imaging unit configured to detect fluorescence emitted from the lower body in response to irradiation of the excitation light,
wherein the illumination unit and the imaging unit are arranged on one side in a first horizontal direction with respect to the imaging region,
the main body portion further includes a restricting portion, and
the restricting portion is disposed along an upper end portion of the imaging region and restricts movement of the subject to the one side in the first horizontal direction.

2. The lymphatic system examination device according to claim 1, further comprising a first plate for luminance calibration, the first plate disposed on an external surface on the imaging unit side in the restricting portion extending in a second horizontal direction perpendicular to the first horizontal direction.

3. The lymphatic system examination device according to claim 1 or 2, further comprising a scale disposed on an external surface on the imaging unit side in the restricting portion extending in a second horizontal direction perpendicular to the first horizontal direction.

4. The lymphatic system examination device according to any one of claims 1 to 3, further comprising a second plate for luminance calibration, the second plate being detachable from the main body portion,
wherein the second plate is disposed in the main body portion so as to be positioned in the imaging region in a state of being in contact with the restricting portion from a side opposite to the imaging unit.

5. The lymphatic system examination device according to any one of claims 1 to 4, further comprising a distance sensor disposed on the one side in the first horizontal direction with respect to the imaging region and configured to measure a distance to the lower body positioned in the imaging region.
